# EUROPEAN PATENT SPECIFICATION

(11) **EP 3 705 023 B1**
(45) Date of publication and mention of the grant of the patent: **10.05.2023**
(21) Application number: 20160517.7
(22) Date of filing: 02.03.2020
(51) Int. Cl.: A61B 1/00, A61B 1/06, A61B 5/00, A61B 5/01, A61B 5/055, A61B 5/107

(54) **ENDOSCOPE APPARATUS, CALIBRATION DEVICE, AND CALIBRATION METHOD**
ENDOSKOPVORRICHTUNG, KALIBRIERUNGSVORRICHTUNG UND KALIBRIERUNGSVERFAHREN
APPAREIL D'ENDOSCOPE, DISPOSITIF D'ÉTALONNAGE ET PROCÉDÉ D'ÉTALONNAGE

(30) Priority: 04.03.2019 JP 2019038172
(43) Date of publication of application: 09.09.2020
(73) Proprietor: FUJIFILM Corporation, Tokyo 106-8620 (JP)
(72) Inventor: UTSUNOMIYA, Daisuke, Kanagawa 258-8538 (JP)
(74) Representative: Klunker IP Patentanwälte PartG mbB

(56) References cited:
- EP-A1- 2 912 994
- EP-A1- 3 698 714
- WO-A1-2018/051679
- JP-A- 2014 090 780
- JP-A- 2018 202 104
- US-A1- 2017 020 627

## Description

### BACKGROUND OF THE INVENTION

### 1. Field of the Invention

The present invention relates to an endoscope apparatus that measures the size of a subject, a calibration device, and a calibration method.

### 2. Description of the Related Art

A distance to a subject, the size of a subject, or the like is acquired in an endoscope apparatus. In, for example, WO2018/051680A, a subject is illuminated with illumination light and spot light for measurement, and a measurement marker used to measure the size of the subject is displayed in a picked-up image so as to correspond to the position of the spot light illuminated on the subject. Further, WO2018/051680A discloses that a relationship between the position of spot light and the size of the subject is calibrated. In WO2018/051680A, a graph paper-shaped chart is illuminated with spot light for measurement, and a relationship between the irradiation position of spot light for measurement and the size of a measurement marker having a specific size is determined from calibration images that are obtained from image pickup at every distance to the chart.

WO2018051679A1 discloses a measurement assistance device, endoscope system, processor for endoscope system, and measurement assistance method. The position of a spot formed by a measurement aid light is measured, information indicating the actual size of a subject is acquired on the basis of the measurement result, and a marker is generated and displayed, and there is therefore no need to gauge a distance, and measurement is easy. By appropriately setting the inclination angle of the measurement aid light, the measurement aid light can be prevented from being outside the field of view of an imaging optical system even when an observation distance is small, and the range of observation distances can be expanded. Furthermore, the optical axis of the measurement aid light has an inclination angle that is not zero degrees with respect to the optical axis of the imaging optical system when projected onto a plane including the optical axis of the imaging optical system, and high measurement precision and high sensitivity to change in the position of the spot with respect to a change in the observation distance are therefore obtained.

JP2014090780 discloses a calibration system having a screen adapted to be moved towards a holder configured to hold an endoscope, wherein by adjusting the distance between the tip portion of the endoscope and the screen, calibration of a scan area can be performed.

### SUMMARY OF THE INVENTION

It is known that a distortion and the like are caused on a subject by an image pickup optical system at the peripheral portion of a picked-up image obtained by the endoscope apparatus. In contrast, in WO2018/051680A, a measurement marker is displayed without the consideration of the influence of a distortion and the like caused by an image pickup optical system. For this reason, there is a case where the size of a subject cannot be accurately measured by a measurement marker in a case where a subject is positioned at the peripheral portion of a picked-up image.

An object of the invention is to provide an endoscope apparatus that can display a measurement marker used to measure the size of a subject in consideration of the influence of a distortion and the like caused by an image pickup optical system, a calibration device, and a calibration method.

In one aspect of the invention, there is provided a calibration device according to claim 1 of the appended claims.

It is preferable that the calibration unit performs representative point-extraction processing for extracting a representative point from the image of the measurement marker of the second display aspect and table-creating processing for creating a representative point data table so that representative point data about the representative point are associated with the irradiation position.

In another aspect of the invention, there is provided a calibration method according to claim 3 of the appended claims.

It is preferable that the calibration step includes a representative point data table-creating step of extracting a representative point from the image of the measurement marker of the second display aspect and creating a representative point data table so that representative point data about the representative point are associated with the irradiation position. It is preferable that the calibration step includes a marker table-creating step of creating a marker table for displaying the image of the measurement marker in the second display aspect by the observation display unit by associating the image of the measurement marker of the second display aspect with the irradiation position. It is preferable that the first display aspect is a size or a shape corresponding to a marker display position and the second display aspect is a size or a shape corresponding to a marker display position.

According to the invention, it is possible to display a measurement marker used to measure the size of a subject in consideration of the influence of a distortion and the like caused by an image pickup optical system.

### BRIEF DESCRIPTION OF THE DRAWINGS

Fig. 1 is a diagram showing the appearance of an endoscope apparatus.
Fig. 2 is a plan view of a distal end part of an endoscope.
Fig. 3 is a block diagram showing the functions of the endoscope apparatus.
Fig. 4 is a block diagram of an auxiliary measurement light-emitting unit.
Fig. 5 is a diagram illustrating a spot SP that is formed on a subject by auxiliary measurement light.
Fig. 6 is a diagram illustrating a relationship between the distal end part of the endoscope and a near end Px, an intermediate vicinity Py, and a far end Pz in a range Rx of an observation distance.
Fig. 7 is a block diagram showing the functions of a signal processing unit.
Fig. 8 is an image diagram showing a spot and a first measurement marker in a case where an observation distance corresponds to the near end Px.
Fig. 9 is an image diagram showing a spot and a first measurement marker in a case where an observation distance corresponds to the intermediate vicinity Py.
Fig. 10 is an image diagram showing a spot and a first measurement marker in a case where an observation distance corresponds to the far end Pz.
Fig. 11 is a diagram illustrating first measurement markers having a cruciform shape, a cruciform shape with gradations, a distorted cruciform shape, a circular-and-cruciform shape, and the shape of a measurement point group.
Fig. 12 is an image diagram showing three markers having the same color and the shapes of concentric circles.
Fig. 13 is an image diagram showing three markers having different colors and the shapes of concentric circles.
Fig. 14 is an image diagram showing markers having the shapes of distorted concentric circles.
Fig. 15 is an image diagram showing a crossing line and gradations.
Fig. 16 is a diagram illustrating a plurality of representative points.
Fig. 17 is a diagram illustrating interpolation processing.
Fig. 18 is a block diagram showing the functions of a calibration device.
Fig. 19 is a diagram illustrating the image of a measurement marker of a first display aspect.
Fig. 20 is a diagram illustrating the image of a measurement marker of a second display aspect.
Fig. 21 is a diagram illustrating representative point-extraction processing.
Fig. 22 is a flowchart showing a calibration method according to an embodiment of the invention.

### DESCRIPTION OF THE PREFERRED EMBODIMENTS

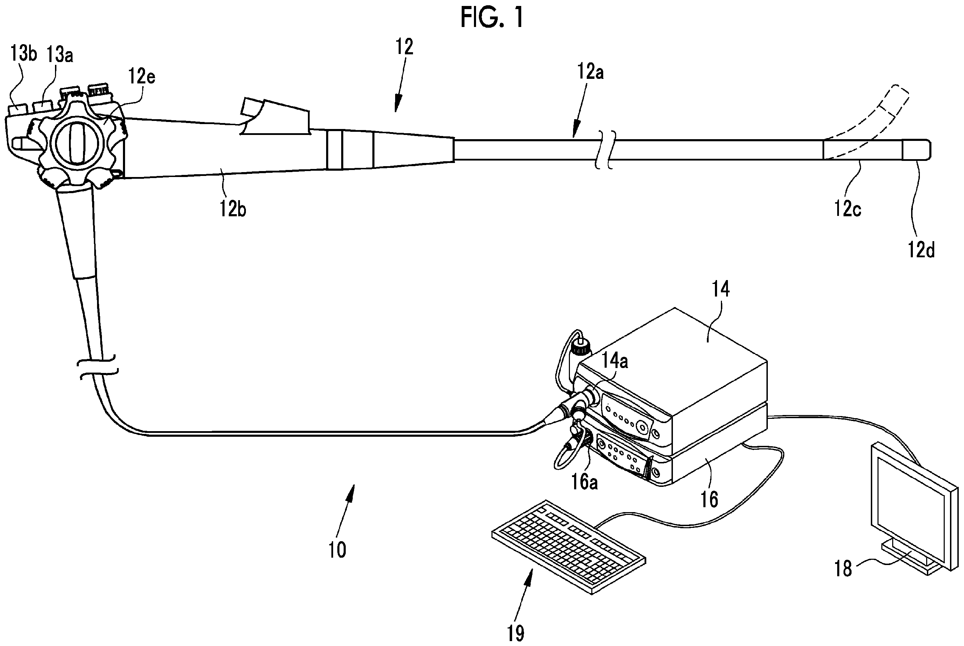

As shown in Fig. 1, an endoscope apparatus 10 includes an endoscope 12, a light source device 14, a processor device 16, an observation display unit 18, and a user interface 19. The endoscope 12 is optically connected to a light source-side connection part 14a of the light source device 14, and is electrically connected to a processor-side connection part 16a of the processor device 16. The processor device 16 is electrically connected to the observation display unit 18 that displays an image. The user interface 19 is connected to the processor device 16, and is used for various setting operations and the like for the processor device 16. The user interface 19 includes a mouse and the like in addition to a keyboard (not shown). Further, the light source-side connection part 14a and the processor-side connection part 16a form an endoscope connection unit.

The endoscope 12 includes an insertion part 12a that is to be inserted into an object to be examined, an operation part 12b that is provided at the proximal end portion of the insertion part 12a, and a bendable part 12c and a distal end part 12d that are provided on the distal end side of the insertion part 12a. The bendable part 12c operates to be bent by the operation of angle knobs 12e of the operation part 12b. The distal end part 12d is made to face in a desired direction by the bending operation of the bendable part 12c.

The endoscope 12 has a normal light observation mode and a length measurement mode, and these two modes are switched by a mode changeover switch 13a that is provided on the operation part 12b of the endoscope 12. The normal light observation mode is a mode where an object to be observed is illuminated with illumination light. In the length measurement mode, an object to be observed is illuminated with illumination light and auxiliary measurement light and a measurement marker to be used to measure the size of the object to be observed or the like is displayed in a picked-up image obtained from the image pickup of the object to be observed. Auxiliary measurement light is light that is used to measure a subject.

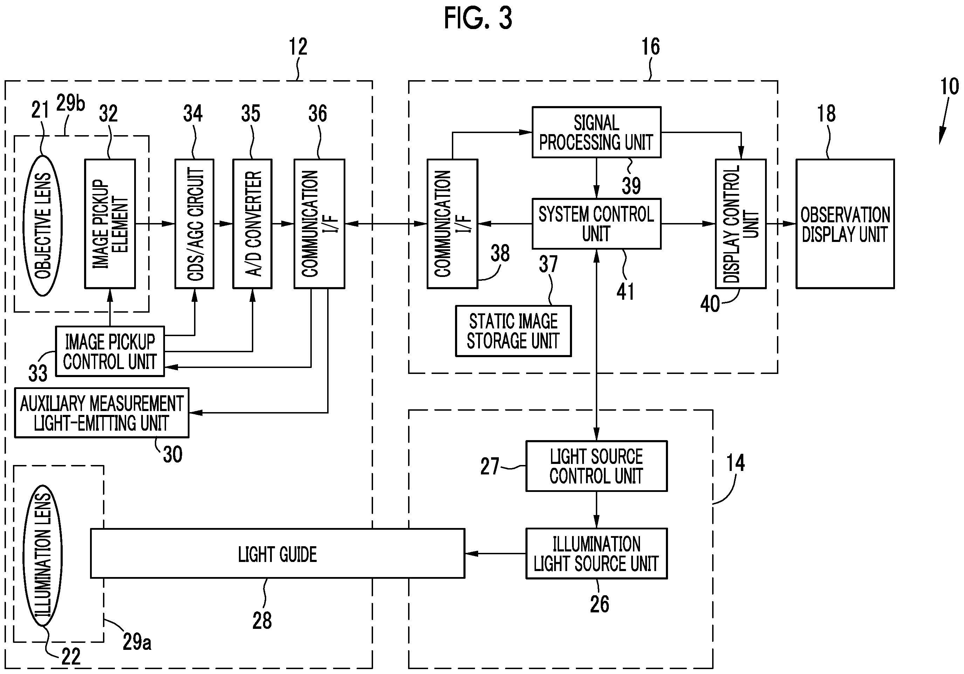

Further, the operation part 12b of the endoscope 12 is provided with a freeze switch 13b that is used to give a static image-acquisition instruction to acquire the static image of a picked-up image. In a case where a user operates the freeze switch 13b, the screen of the observation display unit 18 is frozen and displayed and an alert sound (for example, "beep") informing the acquisition of a static image is generated together. Then, the static images of the picked-up image, which are obtained before and after the operation timing of the freeze switch 13b, are stored in a static image storage unit 37 (see Fig. 3) provided in the processor device 16. Furthermore, it is preferable that measurement information to be described later is also stored together with the static image of the picked-up image in a case where the endoscope 12 is set to the length measurement mode. The static image storage unit 37 is a storage unit, such as a hard disk or a universal serial bus (USB) memory. In a case where the processor device 16 can be connected to a network, the static image of the picked-up image may be stored in a static image storage server (not shown), which is connected to a network, instead of or in addition to the static image storage unit 37.

A static image-acquisition instruction may be given using an operation device other than the freeze switch 13b. For example, a foot pedal may be connected to the processor device 16, and a static image-acquisition instruction may be given in a case where a user operates the foot pedal (not shown) with a foot. A static image-acquisition instruction may be given by a foot pedal that is used to switch a mode. Further, a gesture recognition unit (not shown), which recognizes the gestures of a user, may be connected to the processor device 16, and a static image-acquisition instruction may be given in a case where the gesture recognition unit recognizes a specific gesture of a user. The gesture recognition unit may also be used to switch a mode.

Furthermore, a visual line input unit (not shown), which is provided close to the observation display unit 18, may be connected to the processor device 16, and a static image-acquisition instruction may be given in a case where the visual line input unit recognizes that a user's visual line is in a predetermined region of the observation display unit 18 for a predetermined time or longer. Further, a voice recognition unit (not shown) may be connected to the processor device 16, and a static image-acquisition instruction may be given in a case where the voice recognition unit recognizes a specific voice generated by a user. The voice recognition unit may also be used to switch a mode. Furthermore, an operation panel (not shown), such as a touch panel, may be connected to the processor device 16, and a static image-acquisition instruction may be given in a case where a user makes a specific operation on the operation panel. The operation panel may also be used to switch a mode.

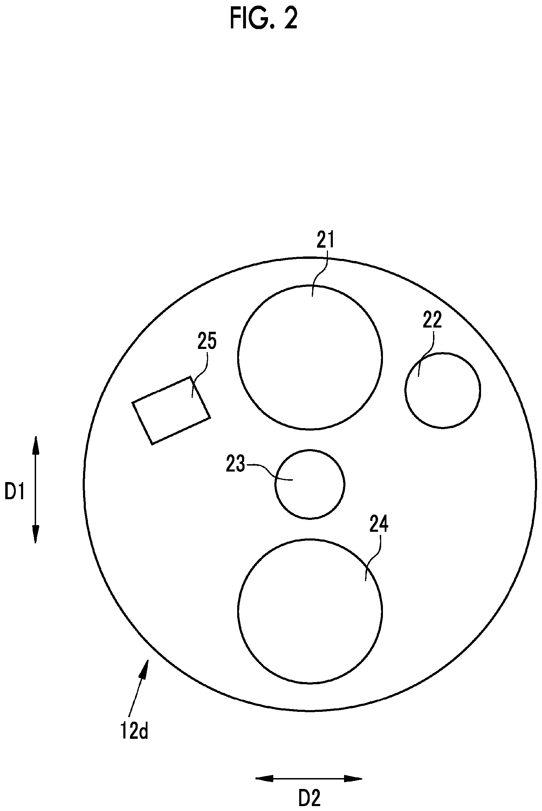

As shown in Fig. 2, the distal end part of the endoscope 12 has a substantially circular shape; and is provided with an objective lens 21 that is positioned closest to a subject among optical members of an image pickup optical system of the endoscope 12, an illumination lens 22 that is used to irradiate a subject with illumination light, an auxiliary measurement lens 23 that is used to illuminate a subject with auxiliary measurement light to be described later, an opening 24 that allows a treatment tool to protrude toward a subject, and an air/water supply nozzle 25 that is used to supply air and water.

An optical axis Ax of the objective lens 21 extends in a direction perpendicular to the plane of paper. A vertical first direction D1 is orthogonal to the optical axis Ax, and a horizontal second direction D2 is orthogonal to the optical axis Ax and the first direction D1. The objective lens 21 and the auxiliary measurement lens 23 are arranged in the first direction D1.

As shown in Fig. 3, the light source device 14 comprises an illumination light source unit 26 and a light source control unit 27. The illumination light source unit 26 generates illumination light that is used to illuminate a subject. Illumination light emitted from the illumination light source unit 26 is incident on a light guide 28, and a subject is irradiated with illumination light through the illumination lens 22. In the illumination light source unit 26, a white light source emitting white light, a plurality of light sources, which includes a white light source and a light source emitting another color light (for example, a blue light source emitting blue light), or the like is used as a light source of illumination light. The light source control unit 27 is connected to a system control unit 41 of the processor device 16. Mixed white light, which is a combination of blue light, green light, and red light, may be used as illumination light. In this case, it is preferable that the illumination lens 22 is optically designed to allow the irradiation range of green light to be wider than the irradiation range of red light.

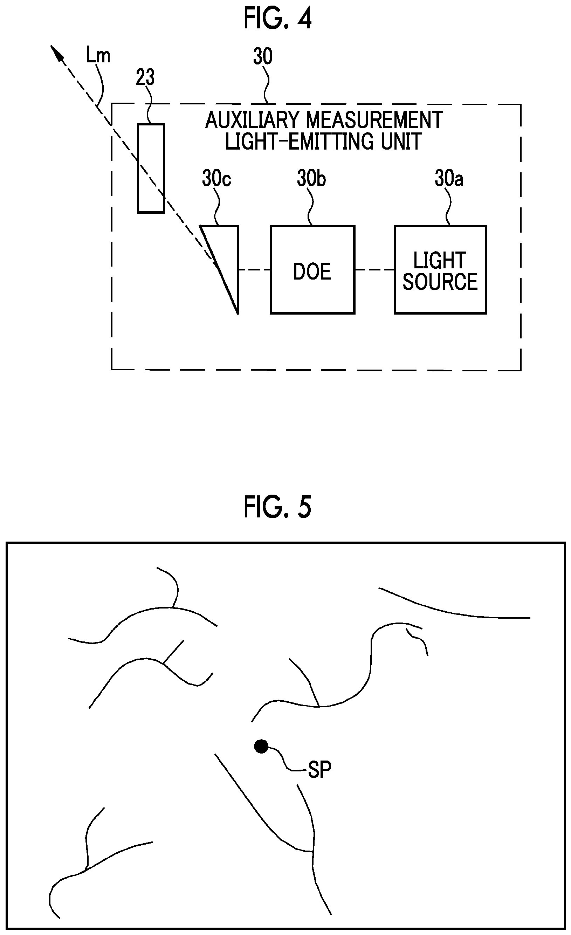

The light source control unit 27 controls the illumination light source unit 26 on the basis of an instruction given from the system control unit 41. The system control unit 41 not only instructs the light source control unit 27 to control a light source but also controls a light source 30a (see Fig. 4) of an auxiliary measurement light-emitting unit 30. In the normal light observation mode, the system control unit 41 performs control to turn on illumination light and to turn off auxiliary measurement light. In the length measurement mode, the system control unit 41 performs control to turn on illumination light and to turn on auxiliary measurement light.

An illumination optical system 29a, an image pickup optical system 29b, and an auxiliary measurement light-emitting unit 30 are provided in the distal end part 12d of the endoscope 12. The illumination optical system 29a includes the illumination lens 22, and an object to be observed is irradiated with light, which is emitted from the light guide 28, through the illumination lens 22. The image pickup optical system 29b includes the objective lens 21 and an image pickup element 32. Light reflected from the object to be observed is incident on the image pickup element 32 through the objective lens 21. Accordingly, the reflected image of the object to be observed is formed on the image pickup element 32.

The image pickup element 32 is a color image pickup sensor, and picks up the reflected image of an object to be examined and outputs image signals. It is preferable that the image pickup element 32 is a charge coupled device (CCD) image pickup sensor, a complementary metal-oxide semiconductor (CMOS) image pickup sensor, or the like. The image pickup element 32 used in the invention is a color image pickup sensor that is used to obtain red images, green images, and red images having three colors of R (red), G (green), and B (blue). The red images are images that are output from red pixels provided with red color filters in the image pickup element 32. The green images are images that are output from green pixels provided with green color filters in the image pickup element 32. The blue images are images that are output from blue pixels provided with blue color filters in the image pickup element 32. The image pickup element 32 is controlled by an image pickup control unit 33.

Image signals output from the image pickup element 32 are transmitted to a CDS/AGC circuit 34. The CDS/AGC circuit 34 performs correlated double sampling (CDS) or auto gain control (AGC) on the image signals that are analog signals. The image signals, which have been transmitted through the CDS/AGC circuit 34, are converted into digital image signals by an analog/digital converter (A/D converter) 35. The digital image signals, which have been subjected to A/D conversion, are input to the processor device 16 through a communication interface (I/F) 36.

The processor device 16 comprises a communication interface (I/F) 38 that is connected to the communication I/F 36 of the endoscope 12, a signal processing unit 39, a display control unit 40, and the system control unit 41. The communication I/F receives the image signals, which are transmitted from the communication I/F 36 of the endoscope 12, and transmits the image signals to the signal processing unit 39. A memory, which temporarily stores the image signals received from the communication I/F 38, is built in the signal processing unit 39, and the signal processing unit 39 processes an image signal group, which is a set of the image signals stored in the memory, to generate the picked-up image. In a case where the endoscope 12 is set to the length measurement mode, the signal processing unit 39 may be adapted to perform structure-emphasis processing for emphasizing structures, such as blood vessels, or color difference-emphasis processing for increasing a color difference between a normal area and a lesion area of the object to be observed on the picked-up image.

The display control unit 40 causes the observation display unit 18 to display the picked-up image that is formed by the signal processing unit 39. The system control unit 41 performs the control of the image pickup element 32 through the image pickup control unit 33 provided in the endoscope 12. The image pickup control unit 33 also performs the control of the CDS/AGC circuit 34 and the A/D converter 35 together with the control of the image pickup element 32.

As shown in Fig. 4, the auxiliary measurement light-emitting unit 30 (auxiliary measurement light source unit) comprises a light source 30a, a diffractive optical element (DOE) 30b, a prism 30c, and the auxiliary measurement lens 23. The light source 30a is to emit light having a color that can be detected by pixels of the image pickup element 32 (specifically visible light), and includes a light-emitting element, such as a laser light source LD (laser diode) or a light emitting diode (LED), and a condenser lens that condenses light emitted from the light-emitting element.

Red laser light having a wavelength in the range of, for example, 600 nm to 650 nm is used in this embodiment as light that is emitted from the light source 30a. However, light having a wavelength in other ranges, for example, green light having a wavelength in the range of, for example, 495 nm to 570 nm may be used. The light source 30a is controlled by the system control unit 41 and emits light on the basis of an instruction given from the system control unit 41. The DOE 30b converts the light, which is emitted from the light source, into auxiliary measurement light that is used to obtain measurement information.

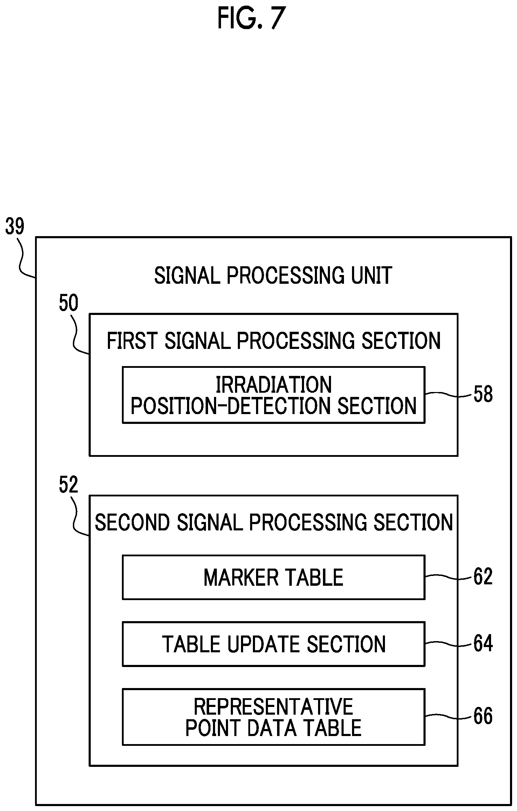

The prism 30c is an optical member that is used to change the travel direction of auxiliary measurement light converted by the DOE 30b. The prism 30c changes the travel direction of auxiliary measurement light so that the auxiliary measurement light crosses the visual field of the image pickup optical system 29b including the objective lens 21 and lens groups. The details of the travel direction of auxiliary measurement light will also be described later. A subject is irradiated with auxiliary measurement light, which is emitted from the prism 30c, through the auxiliary measurement lens 23. In a case where the subject is irradiated with auxiliary measurement light, a spot SP as a circular region is formed on the subject as shown in Fig. 5. The position of the spot SP (the irradiation position of auxiliary measurement light) is detected by an irradiation position-detection section 58 (see Fig. 7), and a measurement marker showing an actual size is set according to the position of the spot SP. The set measurement marker is displayed in the picked-up image. Plural kinds of measurement markers, such as a first measurement marker and a second measurement marker, are included in the measurement marker and an offset measurement marker to be described later as described later, and a measurement marker to be displayed in the picked-up image among the plural kinds of measurement markers can be selected according to a user's instruction. For example, the user interface 19 is used for the user's instruction.

An auxiliary measurement slit formed in the distal end part 12d of the endoscope may be used instead of the auxiliary measurement lens 23. Further, it is preferable that an anti-reflection coating (AR coating) (anti-reflection portion) is provided on the auxiliary measurement lens 23. The reason why the anti-reflection coating is provided as described above is that it is difficult for the irradiation position-detection section 58 to be described later to recognize the position of the spot SP formed on the subject by auxiliary measurement light in a case where auxiliary measurement light is reflected without being transmitted through the auxiliary measurement lens 23 and a ratio of auxiliary measurement light with which a subject is irradiated is reduced.

The auxiliary measurement light-emitting unit 30 has only to be capable of emitting auxiliary measurement light to the visual field of the image pickup optical system 29b. For example, the light source 30a may be provided in the light source device and light emitted from the light source 30a may be guided to the DOE 30b by optical fibers. Further, the prism 30c may not be used and the directions of the light source 30a and the DOE 30b may be inclined with respect to the optical axis Ax so that auxiliary measurement light is emitted in a direction crossing the visual field of the image pickup optical system 29b.

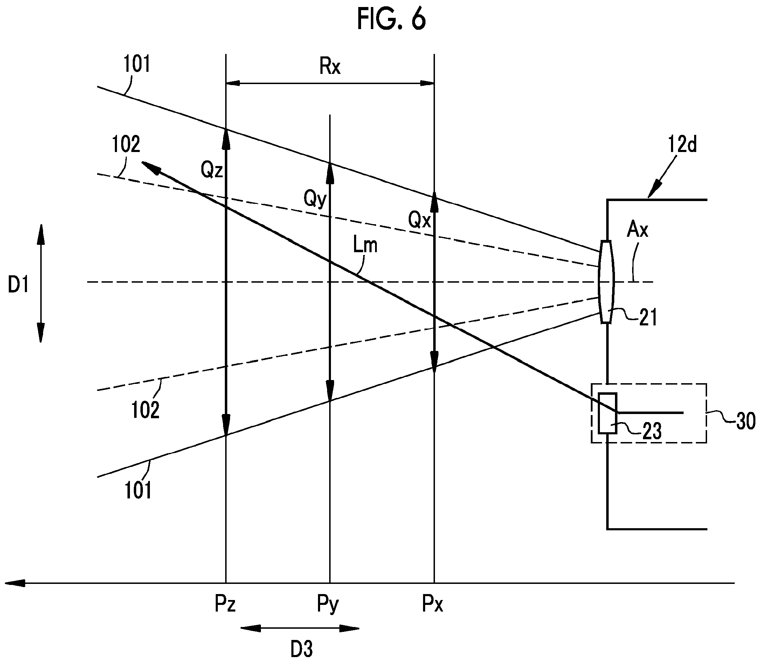

With regard to the travel direction of auxiliary measurement light, auxiliary measurement Lm is emitted in a state where an optical axis Lm of auxiliary measurement light crosses the optical axis Ax of the objective lens 21 as shown in Fig. 6. In a case where a subject can be observed in a range Rx of an observation distance, it is understood that the positions (points where the respective arrows Qx, Qy, and Qz cross the optical axis Ax) of spots SP formed on the subject by auxiliary measurement light in image pickup ranges (shown by arrows Qx, Qy, and Qz) at a near end Px, an intermediate vicinity Py, and a far end Pz of the range Rx are different from each other. The image pickup angle of view of the image pickup optical system 29b is represented by a region between two solid lines 101, and measurement is performed in a central region (a region between two dotted lines 102), in which an aberration is small, of this image pickup angle of view.

Since auxiliary measurement light is emitted in a state where the optical axis Lm of auxiliary measurement light crosses the optical axis Ax as described above, sensitivity to the movement of the position of the spot with respect to a change in the observation distance is high. Accordingly, the size of the subject can be measured with high accuracy. Then, the image of the subject illuminated with auxiliary measurement light is picked up by the image pickup element 32, so that a picked-up image including the spot SP is obtained. In the picked-up image, the position of the spot SP varies depending on a relationship between the optical axis Ax of the objective lens 21 and the optical axis Lm of auxiliary measurement light and an observation distance. The number of pixels showing the same actual size (for example, 5 mm) is increased in the case of a short observation distance, and the number of pixels showing the same actual size (for example, 5 mm) is reduced in the case of a long observation distance.

As shown in Fig. 7, the signal processing unit 39 of the processor device 16 comprises a first signal processing section 50 and a second signal processing section 52 to perform the recognition of the position of the spot SP and the setting of a measurement marker. The first signal processing section 50 detects the position of the spot SP in the picked-up image, and the second signal processing section 52 sets a measurement marker according to the position of the spot SP. In a case where the endoscope 12 is set to the normal light observation mode, the picked-up image of a subject illuminated with illumination light is input to the signal processing unit 39. In a case where the endoscope 12 is set to the length measurement mode, the picked-up image of the subject illuminated with illumination light and auxiliary measurement light is input to the signal processing unit 39. The picked-up image is acquired by the communication I/F 38 (image acquisition unit) connected to the endoscope 12.

The first signal processing section 50 comprises an irradiation position-detection section 58 that detects the irradiation position of the spot SP from the picked-up image. It is preferable that the coordinates of the position of the centroid of the spot SP are acquired in the irradiation position-detection section 58 as the irradiation position of the spot SP.

The second signal processing section 52 sets a first measurement marker as a measurement marker that is used to measure the size of the subject on the basis of the irradiation position of the spot SP, and sets a marker display position where the first measurement marker is to be displayed on the observation display unit 18. The second signal processing section 52 sets a measurement marker image corresponding to an irradiation position with reference to a marker table 62 in which measurement marker images of which display aspects vary depending on the irradiation position of a spot SP and a marker display position and the irradiation positions of a spot are stored in association with each other. For example, the size or shape of a measurement marker image varies depending on the irradiation position of the spot SP and the marker display position. The display of the measurement marker image will be described later. Further, contents stored in the marker table 62 are retained even in a case where the power source of the processor device 16 is turned off. Measurement marker images and irradiation positions are stored in the marker table 62 in association with each other, but distances to subjects (distances between the distal end part 12d of the endoscope 12 and subjects) corresponding to irradiation positions and measurement marker images may be stored in the marker table 62 in association with each other.

Since the image of a measurement marker is required for every irradiation position, the amount of data thereof is increased. Accordingly, considering the point of view of the capacity, activation, processing time, and the like of a memory that can be stored in the endoscope 12, a case where the image of a measurement marker is stored in the processor device 16 is more preferable than a case where the image of a measurement marker is stored in the memory (not shown) provided in the endoscope 12. Further, the image of a measurement marker is created from representative points of a measurement marker image obtained from calibration as described later. However, since extra time is required in a case where a measurement marker image is created from representative points in the length measurement mode, the real-time property of processing deteriorates. For this reason, after the endoscope 12 is connected to the endoscope connection unit and a measurement marker image is created from representative points to update the marker table 62 once, a measurement marker is not created from representative points and a measurement marker image is displayed using the updated marker table 62. Furthermore, in an emergency where it is difficult to superimpose and display images, instead of a measurement marker image to be superimposed on and displayed in a specific image, a reference marker for determining the size of a marker from a relationship between the irradiation position of the spot SP and the number of pixels corresponding to the actual size of a subject is displayed in the specific image by the second signal processing section 52.

Further, the second signal processing section 52 comprises a table update section 64 that updates the marker table 62 in a case where the endoscope 12 is connected to the endoscope connection unit. The reason why the marker table 62 is adapted to be capable of being updated as described above is that a positional relationship between the optical axis Lm of auxiliary measurement light and the image pickup optical system 29b varies depending on the model and the serial number of the endoscope 12 and the display aspect of a measurement marker image is also changed accordingly. A representative point data table 66 in which representative point data about representative points extracted from a measurement marker image and irradiation positions are stored in association with each other is used in the table update section 64. The details of the table update section 64 and the representative point data table 66 will be described later. Distances to subjects (distances between the distal end part 12d of the endoscope 12 and subjects) corresponding to irradiation positions and the representative point data may be stored in the representative point data table 66 in association with each other.

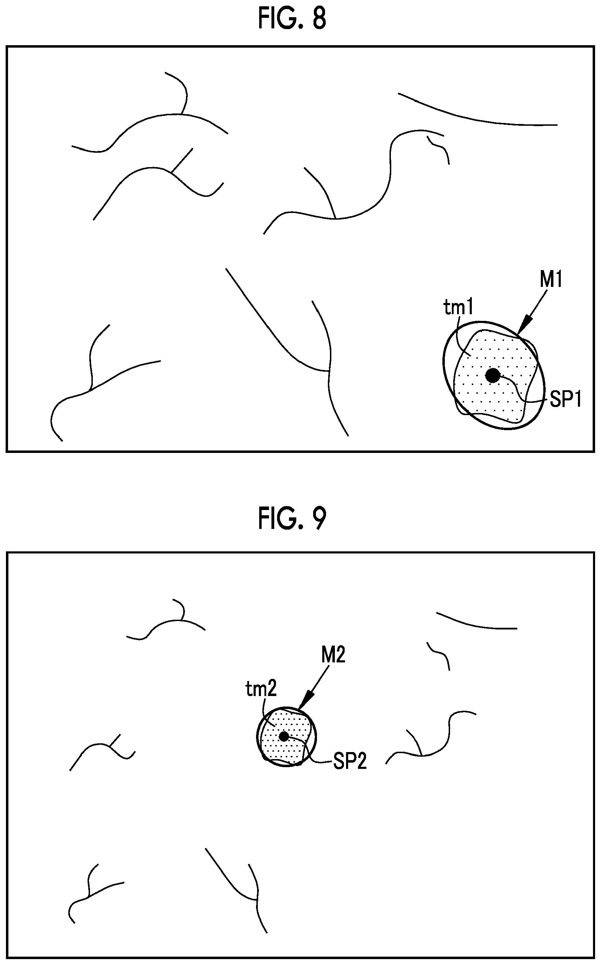

The display control unit 40 performs control where the display aspect of a measurement marker varies according to the irradiation position of the spot SP and a marker display position in a case where a specific image in which the measurement marker is superimposed on a picked-up image is to be displayed on the observation display unit 18. Specifically, the display control unit 40 causes the observation display unit 18 to display the specific image, on which the first measurement marker is superimposed, so that the spot SP is positioned at the center of the specific image. For example, a circular measurement marker is used as the first measurement marker. In this case, as shown in Fig. 8, a marker M1, which shows an actual size of 5 mm (a horizontal direction and a vertical direction of the picked-up image), is displayed at the center of a spot SP1 formed on a tumor tm1 of a subject in a case where an observation distance is close to the near end Px.

Since the marker display position of the marker M1 is positioned at the peripheral portion of the picked-up image that is affected by a distortion and the like caused by the image pickup optical system 29b, the marker M1 is formed in an elliptical shape due to the influence of the distortion and the like. Since the above-mentioned marker M1 substantially coincides with the range of the tumor tm1, the size of the tumor tm1 can be measured as about 5 mm. A spot is not displayed and only the first measurement marker may be displayed in the picked-up image.

Further, as shown in Fig. 9, a marker M2, which shows an actual size of 5 mm (the horizontal direction and the vertical direction of the picked-up image), is displayed at the center of a spot SP2 formed on a tumor tm2 of a subject in a case where an observation distance is close to the intermediate vicinity Py. Since the marker display position of the marker M2 is positioned at the central portion of the picked-up image that is not easily affected by a distortion and the like caused by the image pickup optical system 29b, the marker M2 is formed in a circular shape without being affected by the distortion and the like.

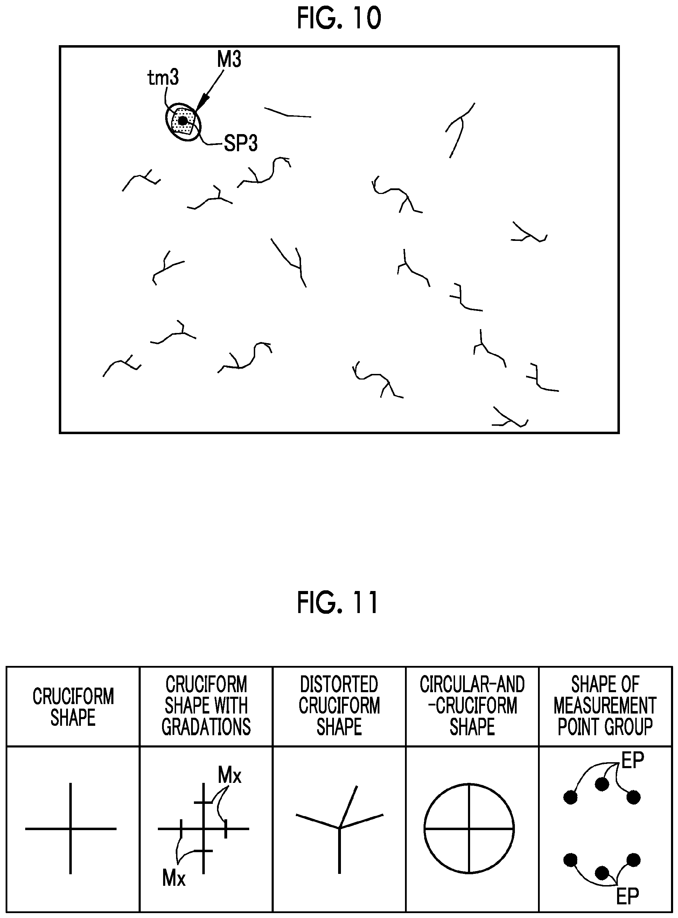

Furthermore, as shown in Fig. 10, a marker M3, which shows an actual size of 5 mm (the horizontal direction and the vertical direction of the picked-up image), is displayed at the center of a spot SP3 formed on a tumor tm3 of a subject. Since the marker display position of the marker M3 is positioned at the peripheral portion of the picked-up image that is affected by a distortion caused by the image pickup optical system 29b, the marker M3 is formed in an elliptical shape due to the influence of the distortion and the like. As shown in Figs. 8 to 10 having been described above, the size of the first measurement marker corresponding to the same actual size of 5 mm is reduced with an increase in an observation distance. Further, the shape of the first measurement marker also varies depending on the marker display position due to the influence of a distortion caused by the image pickup optical system 29b.

In Figs. 8 to 10, the center of the spot SP and the center of the marker are displayed to coincide with each other. However, the first measurement marker may be displayed at a position away from the spot SP in a case where there is no problem in measurement accuracy. Even in this case, it is preferable that the first measurement marker is displayed near the spot. Furthermore, the distorted first measurement marker is not displayed, and the distortion of the picked-up image may be corrected so that an undistorted first measurement marker may be displayed in a corrected picked-up image.

Further, the first measurement marker corresponding to the actual size of the subject, which is 5 mm, is displayed in Figs. 8 to 10, but the actual size of the subject may be set to any value (for example, 2 mm, 3 mm, 10 mm, or the like) according to an object to be observed or the purpose of observation. Furthermore, the first measurement marker has a substantially circular shape in Figs. 8 to 10, but may have a cruciform shape where a vertical line and a horizontal line cross each other as shown in Fig. 11. Moreover, the first measurement marker may have a cruciform shape with gradations where gradations Mx are given to at least one of a vertical line or a horizontal line of a cruciform shape. Further, the first measurement marker may have a distorted cruciform shape of which at least one of a vertical line or a horizontal line is inclined. Furthermore, the first measurement marker may have a circular-and-cruciform shape where a cruciform shape and a circle are combined with each other. In addition, the first measurement marker may have the shape of a measurement point group where a plurality of measurement points EP corresponding to an actual size from a spot are combined with each other. Further, one first measurement marker may be displayed or a plurality of first measurement markers may be displayed, and the color of the first measurement marker may be changed according to an actual size.

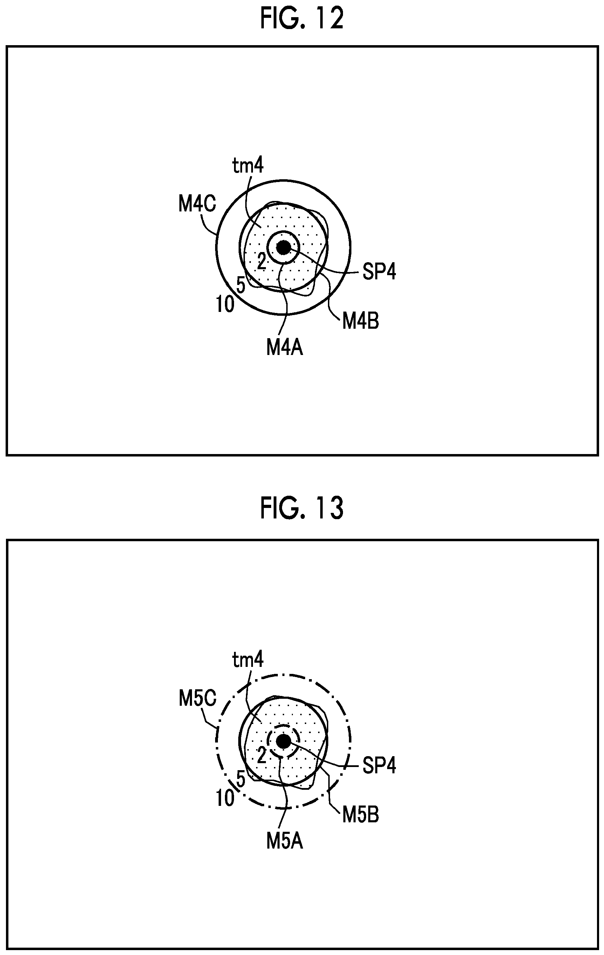

As shown in Fig. 12, three concentric circular markers M4A, M4B, and M4C having different sizes (of which diameters are 2 mm, 5mm, and 10 mm) may be displayed in the picked-up image as the first measurement marker so that a spot SP4 formed on the tumor tm4 is positioned at the centers of the markers. Since a plurality of markers are displayed in the case of the three concentric circular markers, time and effort required to switch a marker can be saved and measurement can be performed even in a case where a subject has a non-linear shape. In a case where a plurality of concentric circular markers are to be displayed so that a spot is positioned at the centers of the concentric circular markers, a size and a color are not designated for each marker and combinations of a plurality of conditions may be prepared in advance and one can be selected from these combinations.

In Fig. 12, all the three concentric circular markers are displayed with the same color (black). However, in a case where a plurality of concentric circular markers are to be displayed, a plurality of color concentric circular markers of which colors are different from each other may be used. As shown in Fig. 13, a marker M5A is displayed by a dotted line representing a red color, a marker M5B is displayed by a solid line representing a blue color, and a marker M5C is displayed by a one-dot chain line representing a white color. Since identifiability can be improved in a case where the colors of the markers are different from each other in this way, measurement can be easily performed.

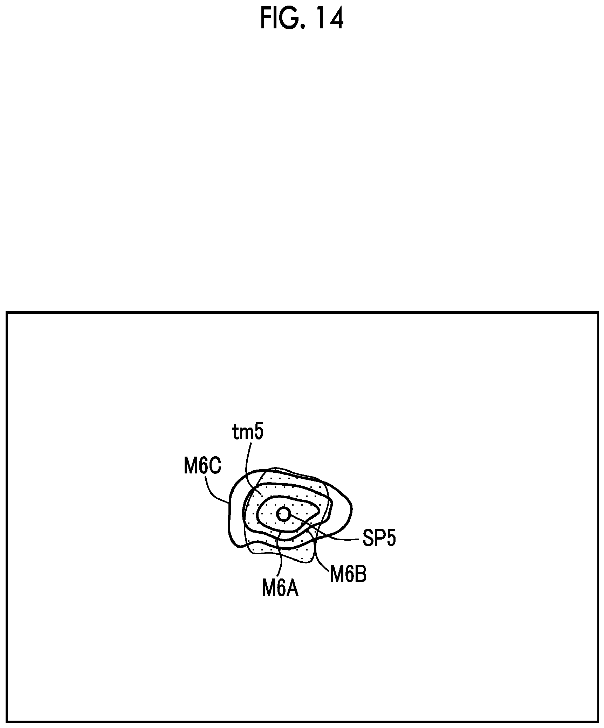

Further, as shown in Fig. 14, a plurality of distorted concentric circular markers, which are distorted from the respective concentric circles, may be used as the first measurement marker other than the plurality of concentric circular markers. In this case, distorted concentric circular markers M6A, M6B, and M6C are displayed in the picked-up image so that a spot SP5 formed on a tumor tm5 is positioned at the centers of the distorted concentric circular markers.

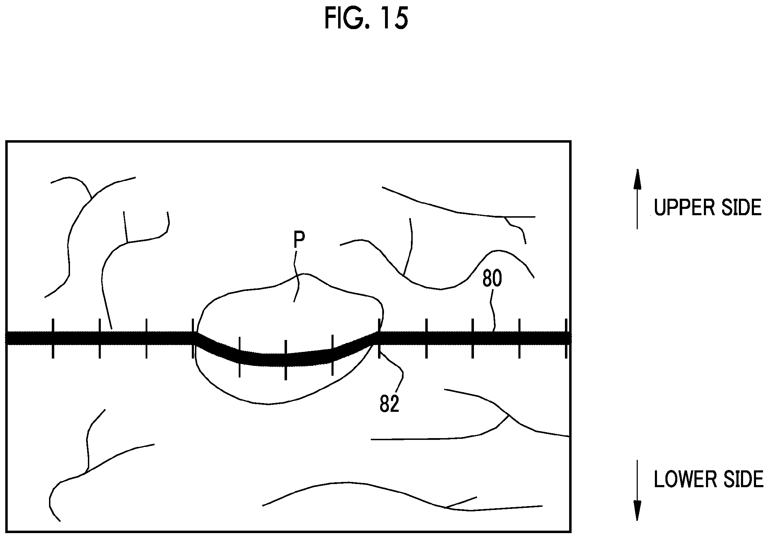

Light that forms a spot in a case where a subject is irradiated with the light is used as auxiliary measurement light, but other light may be used. For example, planar auxiliary measurement light that forms a crossing line 80 on a subject as shown in Fig. 15 in a case where the subject is irradiated with light may be used. In this case, a second measurement marker that includes the crossing line 80 and gradations 82 formed on the crossing line and serving as an index of the size of the subject (for example, a polyp P) is generated as a measurement marker. In a case where planar auxiliary measurement light is used, the irradiation position-detection section 58 detects the position of the crossing line 80 (the irradiation position of auxiliary measurement light). An observation distance is shorter as the crossing line 80 is positioned closer to the lower side, and an observation distance is longer as the crossing line 80 is positioned closer to the upper side. For this reason, an interval between the gradations 82 is larger as the crossing line 80 is positioned closer to the lower side, and an interval between the gradations 82 is smaller as the crossing line 80 is positioned closer to the upper side.

The details of the table update section 64 and the representative point data table 66 will be described. The table update section 64 creates a measurement marker image, which corresponds to the model and/or the serial number of the endoscope 12, with reference to the representative point data table 66 and updates the marker table 62 in a case where the endoscope 12 is connected to the endoscope connection unit.

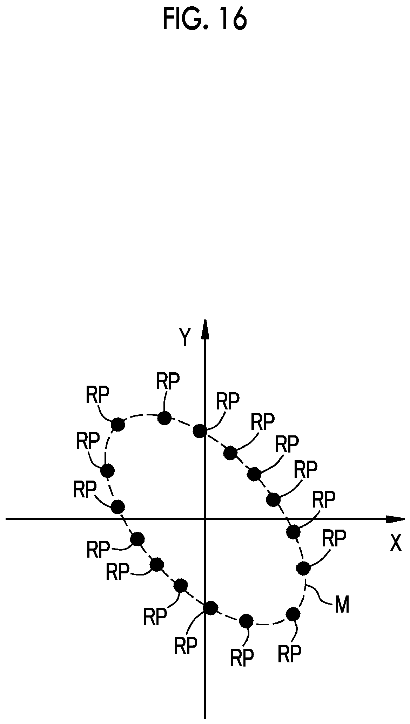

Representative point data about the representative points of a measurement marker image, which is obtained during calibration, and the irradiation positions of a spot SP are stored in the representative point data table 66 in association with each other. The representative point data table 66 is created by a calibration method to be described later. As shown in Fig. 16, coordinate information (X-coordinates and Y-coordinates) of representative points RP, which are some point extracted from a circular marker image M serving as a measurement marker image, are included as the representative point data. The representative point data stored in the representative point data table 66 are data in a case where a positional relationship between the optical axis Lm of auxiliary measurement light and the image pickup optical system 29b is a default positional relationship.

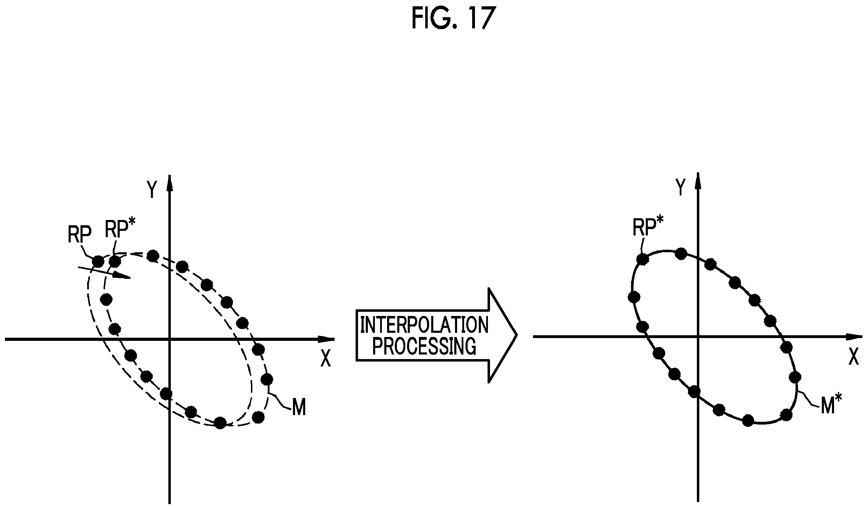

The table update section 64 acquires information about a positional relationship between the optical axis Lm of auxiliary measurement light and the image pickup optical system 29b in a case where the endoscope 12 is connected to the endoscope connection unit; and updates the marker table 62 by using the positional relationship and the representative point data table 66. Specifically, the table update section 64 calculates difference values of the coordinate information of the representative points RP from a difference between the default positional relationship and the positional relationship between the optical axis Lm of auxiliary measurement light and the image pickup optical system 29b of the endoscope 12 connected to the endoscope connection unit. Then, as shown in Fig. 17, the table update section 64 creates a measurement marker image M* on the basis of representative points RP* that are obtained in a case where the coordinates of the default representative points RP are shifted by the calculated difference values. It is preferable that interpolation processing for connecting the representative points RP^{∗} is performed to create the measurement marker image. The created measurement marker image having been subjected to the interpolation processing is associated with irradiation positions by the table update section 64. Accordingly, the update of the marker table 62 is completed. In Fig. 17, only some of the representative points RP and RP* are denoted by reference characters.

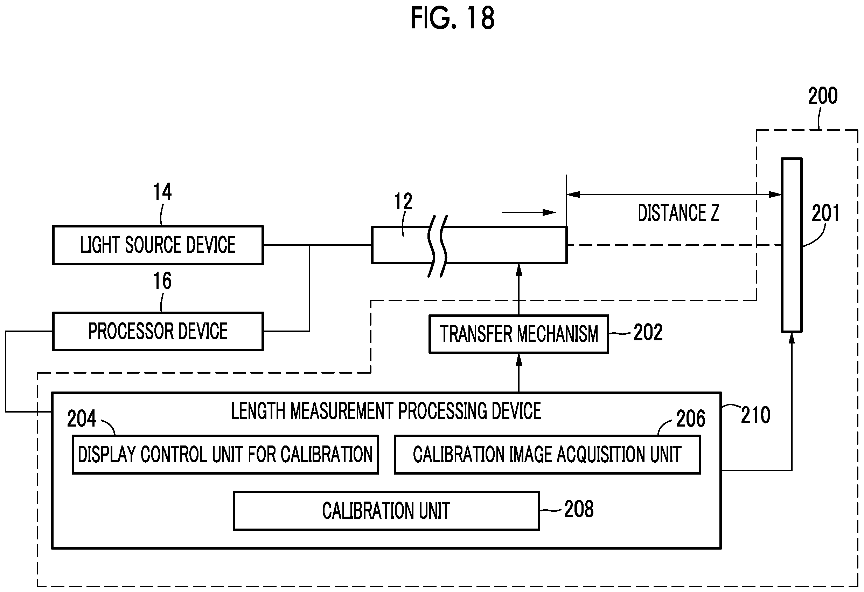

A calibration method according to an embodiment of the invention used to create the representative point data table 66 will be described below. As shown in Fig. 18, the calibration method according to the embodiment of the invention is performed by a calibration device 200. The calibration device 200 comprises a display unit 201 for calibration, a transfer mechanism 202, a display control unit 204 for calibration, a calibration image acquisition unit 206, and a calibration unit 208. The display control unit 204 for calibration, the calibration image acquisition unit 206, and the calibration unit 208 are provided in a length measurement processing device 210. The length measurement processing device 210 is electrically connected to the processor device 16, the display unit 201 for calibration, and the transfer mechanism 202.

The transfer mechanism 202 includes a holding unit (not shown) that holds the distal end part 12d of the endoscope 12 toward the display unit 201 for calibration, and changes a distance Z between the distal end part 12d of the endoscope 12 and the display unit 201 for calibration by transferring the holding unit at a specific interval. In a case where the calibration method is to be performed, the display surface of the display unit 201 for calibration is irradiated with auxiliary measurement light from the distal end part 12d of the endoscope 12. In a case where the distance Z is longer, the irradiation position of auxiliary measurement light is positioned closer to the lower side of the display surface of the display unit 201 for calibration. As the distance Z is reduced, the irradiation position of auxiliary measurement light is positioned closer to the upper side of the display surface of the display unit 201 for calibration.

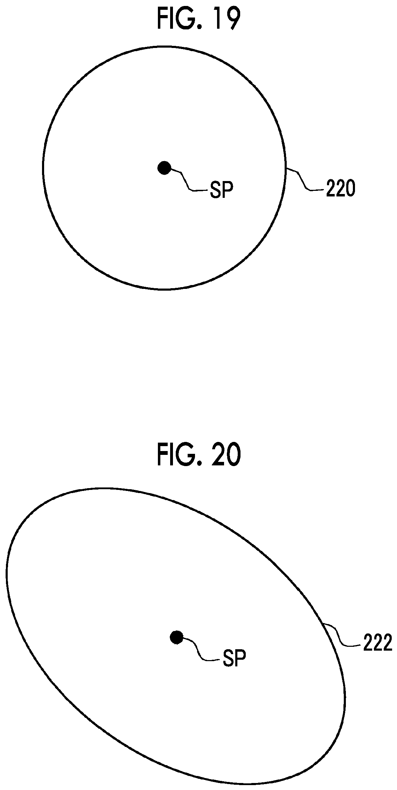

Whenever the distance Z is changed by the transfer mechanism 202, the display control unit 204 for calibration causes the display unit 201 for calibration to display the image of a measurement marker of a first display aspect, which is not affected by the image pickup optical system 29b, at the irradiation position of auxiliary measurement light. Since the influence of a distortion and the like caused by the image pickup optical system 29b is not considered in the image of the measurement marker of the first display aspect, the image is not displayed with a size, a shape, or the like corresponding to a marker display position in a case where the image is to be displayed by the observation display unit 18. Accordingly, in a case where the measurement marker has a circular shape, the image 220 of the measurement marker of the first display aspect is displayed in a circular shape at the peripheral portion of the picked-up image (that is likely to be affected by a distortion) as shown in Fig. 19. In a case where the distance Z is divided into n pieces (n is a natural number), the measurement marker of the first display aspect is displayed n times whenever the distance Z is changed.

The calibration image acquisition unit 206 acquires a calibration image that is obtained in a case where the image of the measurement marker of the first display aspect displayed on the display unit 201 for calibration is picked up by the endoscope 12. The calibration image is acquired in a case where the endoscope 12 picks up an image whenever the distance Z is changed, that is, whenever the measurement marker of the first display aspect is displayed. For example, in a case where the measurement marker of the first display aspect is displayed n times, n calibration images are acquired.

The image of a measurement marker of a second display aspect, which is affected by the image pickup optical system 29b, is included in the calibration image at the irradiation position of auxiliary measurement light. Since the influence of a distortion and the like caused by the image pickup optical system 29b are considered in the image of the measurement marker of the second display aspect, the image is displayed with a size, a shape, or the like corresponding to a marker display position. Accordingly, in a case where the measurement marker has a circular shape, as shown in Fig. 20, the image 222 of the measurement marker of the second display aspect is displayed in an elliptical shape at the peripheral portion of the picked-up image due to the influence of the distortion and the like.

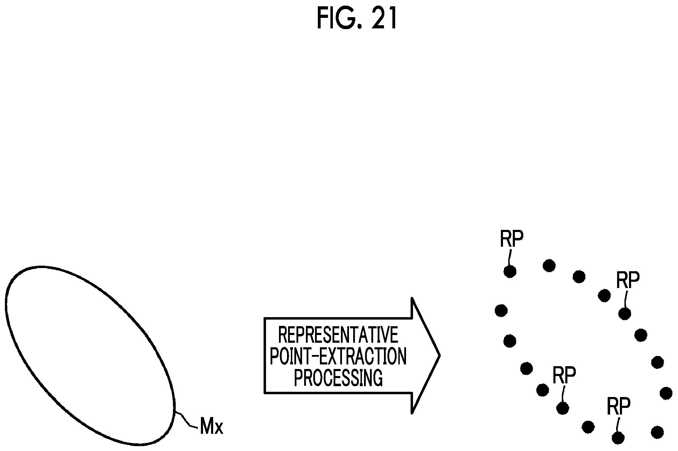

The calibration unit 208 performs calibration related to the display of the measurement marker on the observation display unit 18 on the basis of the calibration image that is acquired by the calibration image acquisition unit 206. Specifically, the calibration unit 208 performs: representative point-extraction processing for extracting representative points from the image of the measurement marker of the second display aspect included in the calibration image; and table-creating processing for creating a representative point data table so that representative point data about the representative points are associated with an irradiation position at a timing when the calibration image is acquired. For example, in a case where the image of the measurement marker of the second display aspect corresponds to an elliptical marker Mx as shown in Fig. 21, a plurality of representative points RP are extracted from the marker Mx by the representative point-extraction processing. Then, the coordinates of the plurality of representative points are used as representative point data, and the representative point data table is created so that the representative point data are associated with an irradiation position at a timing when the calibration image is acquired. The created representative point data table is sent to the processor device 16 and is stored in the representative point data table 66.

In this embodiment, the representative points extracted from the image of the measurement marker of the second display aspect are stored in the form of the representative point data table by the calibration method to reduce the capacity of a memory as much as possible. However, in a case where there is the extra capacity of the memory, a marker table may be created so that the image of the measurement marker of the second display aspect included in the calibration image is associated with an irradiation position at a timing when the calibration image is acquired. In this case, the created marker table is sent to the processor device 16 and is stored in the marker table 62.

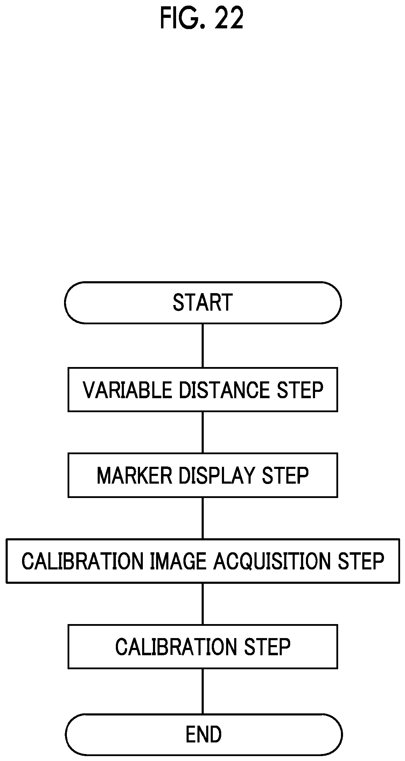

Next, a series of flows of the calibration method according to the embodiment of the invention will be described with reference to a flowchart shown in Fig. 22. The distal end part 12d of the endoscope 12 is set in the holding unit of the transfer mechanism 202. The transfer mechanism 202 changes a distance Z between the distal end part 12d of the endoscope 12 and the display unit 201 for calibration by transferring the holding unit at a specific interval (variable distance step). At this time, the display surface of the display unit 201 for calibration is irradiated with auxiliary measurement light from the distal end part 12d of the endoscope 12. Then, whenever the distance Z is changed by the transfer mechanism 202, the display control unit 204 for calibration causes the display unit 201 for calibration to display the image of the measurement marker of the first display aspect, which is not affected by the image pickup optical system 29b, at the irradiation position of auxiliary measurement light (marker display step).

Next, the calibration image acquisition unit 206 acquires a calibration image that is obtained in a case where the image of the measurement marker of the first display aspect displayed on the display unit 201 for calibration is picked up by the endoscope 12 (calibration image acquisition step). The image of the measurement marker of the second display aspect, which is affected by the image pickup optical system 29b, is included in the calibration image. The calibration unit 208 performs calibration related to the display of the measurement marker on the observation display unit 18 on the basis of the calibration image that is acquired by the calibration image acquisition unit 206 (calibration step).

Specifically, a representative point data table-creating step is performed in the calibration step. In the representative point data table-creating step, representative points are extracted from the image of the measurement marker of the second display aspect included in the calibration image and a representative point data table is created so that representative point data about the representative points are associated with an irradiation position at a timing when the calibration image is acquired. Further, a marker table-creating step may be performed in the calibration step. In the marker table-creating step, a marker table is created so that the image of the measurement marker of the second display aspect included in the calibration image is associated with an irradiation position at a timing when the calibration image is acquired.

A processing unit and a control unit, which are related to the length measurement mode, among the signal processing unit 39 and the display control unit 40 are provided in the processor device 16 in the embodiment. However, a processing unit and a control unit, which are related to the length measurement mode, may be provided in an external length measurement processing device (see Fig. 18) separate from the processor device 16. In this case, the length measurement processing device and the processor device are electrically connected to each other, and data, such as the picked-up image output from the processor device 16, are sent to the length measurement processing device. A specific image, which has been processed by the length measurement processing device, and the like are displayed on a display unit connected to the length measurement processing device.

In the embodiment, the hardware structures of processing units, which perform various kinds of processing, such as the signal processing unit 39, the display control unit 40, the system control unit 41, the first signal processing section 50, the second signal processing section 52, the irradiation position-detection section 58, the marker table 62, the table update section 64, the representative point data table 66, the display control unit 204 for calibration, the calibration image acquisition unit 206, the calibration unit 208, and the length measurement processing device 210, are various processors to be described later. Various processors include: a central processing unit (CPU) that is a general-purpose processor functioning as various processing units by executing software (program); a programmable logic device (PLD) that is a processor of which the circuit configuration can be changed after manufacture, such as a field programmable gate array (FPGA); a dedicated electrical circuit that is a processor having circuit configuration designed exclusively to perform various kinds of processing; and the like.

One processing unit may be formed of one of these various processors, or may be formed of a combination of two or more same kind or different kinds of processors (for example, a plurality of FPGAs, or a combination of a CPU and an FPGA). Further, a plurality of processing units may be formed of one processor. As an example where a plurality of processing units are formed of one processor, first, there is an aspect where one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined.

### Explanation of References

10: endoscope apparatus
12: endoscope
12a: insertion part
12b: operation part
12c: bendable part
12d: distal end part
12e: angle knob
13a: mode changeover switch
13b: freeze switch
14: light source device
16: processor device
18: observation display unit
19: user interface
21: objective lens
22: illumination lens
23: auxiliary measurement lens
24: opening
25: air/water supply nozzle
26: illumination light source unit
27: light source control unit
28: light guide
29a: illumination optical system
29b: image pickup optical system
30: auxiliary measurement light-emitting unit
30a: light source
30b: DOE
30c: prism
32: image pickup element
33: image pickup control unit
34: CDS/AGC circuit
36: communication I/F
37: static image storage unit
38: communication I/F
39: signal processing unit
40: display control unit
41: system control unit
50: first signal processing section
52: second signal processing section
58: irradiation position-detection section
62: marker table
64: table update section
66: representative point data table
80: crossing line
101: solid line
102: dotted line
200: calibration device
201: display unit for calibration
202: transfer mechanism
204: display control unit for calibration
206: calibration image acquisition unit
208: calibration unit
210: length measurement processing device
220: image of measurement marker of first display aspect
222: image of measurement marker of second display aspect
M1, M2, M3: cruciform marker
tm1, tm2, tm3, tm4, tm5: tumor
SP: spot
SP1, SP2, SP3, SP4, SP5: spot
M, M^{∗}: measurement marker image
M4A, M4B, M4C, M5A, M5B, M5C: concentric circular marker
M6A, M6B, M6C: distorted concentric circular marker
P: polyp
RP, RP*: representative point

## Claims

1. A calibration device (200) connectable to an endoscope apparatus (10) including an endoscope (12) that emits auxiliary measurement light and picks up an image of a subject illuminated with the auxiliary measurement light through an image pickup optical system (29b), wherein the auxiliary measurement light crosses the optical axis of the image pickup optical system (29b), the calibration device (200) comprising:
a transfer mechanism (202) configured to hold a distal end part (12d) of the endoscope (12) toward a display unit (201) for calibration and configured to change a distance between the distal end part of the endoscope (12) and the display unit for calibration (201);
a display control unit (40) for calibration configured to cause the display unit for calibration (201) to display a measurement marker of a first display aspect (220), which is not affected by the image pickup optical system (29b), at an irradiation position to which the auxiliary measurement light is applied whenever the distance is changed;
a marker image acquisition unit configured to acquire a calibration image obtained in a case where an image of the measurement marker of the first display aspect (220) displayed on the display unit for calibration (201) is picked up by the endoscope (12), the calibration image including an image of a measurement marker of a second display aspect (222) affected by the image pickup optical system (29b); and
a calibration unit (208) configured to perform calibration related to display of the measurement marker on an observation display unit (18) on the basis of the calibration image.

2. The calibration device according to claim 1,
wherein the calibration unit (208) performs representative point-extraction processing for extracting a representative point from the image of the measurement marker of the second display aspect (222), and table-creating processing for creating a representative point data table so that representative point data about the representative point are associated with the irradiation position.

3. A calibration method for an endoscope apparatus (10) including an endoscope (12) that emits auxiliary measurement light and picks up an image of a subject illuminated with the auxiliary measurement light through an image pickup optical system (29b), wherein the auxiliary measurement light crosses the optical axis of the image pickup optical system (29b), the calibration method comprising:
a variable distance step of holding a distal end part (12d) of the endoscope (12) toward a display unit (201) for calibration and changing a distance between the distal end part of the endoscope and the display unit for calibration (201) by a transfer mechanism;
a marker display step of causing the display unit for calibration (201) to display a measurement marker of a first display aspect (220), which is not affected by the image pickup optical system (29b), at an irradiation position to which the auxiliary measurement light is applied whenever the distance is changed;
a marker image acquisition step of acquiring a calibration image obtained in a case where an image of the measurement marker of the first display aspect displayed on the display unit for calibration is picked up by the endoscope (12), the calibration image including an image of a measurement marker of a second display aspect (222) affected by the image pickup optical system (29b); and
a calibration step of performing calibration related to display of the measurement marker on an observation display unit (18) on the basis of the image of the measurement marker of the second display aspect (222).

4. The calibration method according to claim 3,
wherein the calibration step includes a representative point data table-creating step of extracting a representative point from the image of the measurement marker of the second display aspect (222) and creating a representative point data table so that representative point data about the representative point are associated with the irradiation position.

5. The calibration method according to claim 3,
wherein the calibration step includes a marker table-creating step of creating a marker table for displaying the image of the measurement marker in the second display aspect (222) by the observation display unit (18) by associating the image of the measurement marker of the second display aspect (222) with the irradiation position.

6. The calibration method according to any one of claims 3 to 5,
wherein the first display aspect is a size or a shape corresponding to a marker display position, and the second display aspect is a size or a shape corresponding to a marker display position.

## Patentansprüche

1. Kalibriervorrichtung (200), verbindbar mit einer Endoskopvorrichtung (10), die ein Endoskop (12) enthält, das Hilfsmesslicht emittiert und ein Bild eines Subjekts, das mit dem Hilfsmesslicht beleuchtet wird, über eine Bildaufnahmeoptik (29b) aufnimmt, wobei das Hilfsmesslicht die optische Achse der Bildaufnahmeoptik (29b) kreuzt, umfassend:
einen Transfermechanismus (202), konfiguriert zum Halten eines distalen Endteils (12d) des Endoskops (12) in Richtung einer Anzeigeeinheit (201) zum Kalibrieren, und konfiguriert zum Ändern eines Abstands zwischen dem distalen Endteil des Endoskops (12) und der Anzeigeeinheit zum Kalibrieren (201);
eine Anzeigesteuereinheit (40) zum Kalibrieren, konfiguriert zum Veranlassen der Anzeigeeinheit zum Kalibrieren (201), eine Messmarkierung eines ersten Anzeigeaspekts (220) anzuzeigen, der nicht von der Bildaufnahmeoptik (29b) beeinflusst ist, an einer Bestrahlungsposition, an der das Hilfsmesslicht aufgebracht wird, wann immer der Abstand geändert wird;
eine Markierungsbild-Erfassungseinheit, konfiguriert zum Erfassen eines Kalibrierbilds, welches dann erhalten wird, wenn ein Bild der Messmarkierung des ersten Anzeigeaspekts (220), das auf der Anzeigeeinheit zur Kalibrierung (201) angezeigt wird, von dem Endoskop (12) aufgenommen wird, wobei das Kalibrierbild ein Bild einer Messmarkierung eines zweiten Anzeigeaspekts (222) enthält, der von der Bildaufnahmeoptik (29b) beeinflusst wird; und
eine Kalibriereinheit (208), konfiguriert zum Ausführen einer Kalibrierung bezüglich der Anzeige der Messmarkierung auf einer Beobachtungs-Anzeigeeinheit (18) auf der Grundlage des Kalibrierbilds.

2. Kalibriervorrichtung nach Anspruch 1,
bei der die Kalibriereinheit (208) eine repräsentative Punktextraktionsverarbeitung zum Extrahieren eines repräsentativen Punkts aus dem Bild der Messmarkierung des zweiten Anzeigeaspekts (222) ausführt, ferner eine Tabellenerzeugungsverarbeitung zum Erzeugen einer repräsentativen Punktdatentabelle, so dass repräsentative Punktdaten bezüglich des repräsentativen Punkts mit der Bestrahlungsposition in Verbindung gebracht werden.

3. Kalibrierverfahren für eine Endoskopvorrichtung (10), die ein Endoskop (12) enthält, die Hilfsmesslicht emittiert und ein Bild eines mit dem Hilfsmesslicht beleuchteten Subjekts über eine Bildaufnahmeoptik (29b) aufnimmt, wobei das Hilfsmesslicht eine optische Achse der Bildaufnahmeoptik (29b) kreuzt, umfassend:
einen veränderlichen Abstandsschritt zum Halten eines distalen Endteils (12d) des Endoskops (12) in Richtung einer Anzeigeeinheit (201) zum Kalibrieren, und zum Ändern einer Distanz zwischen dem distalen Endteil des Endoskops und der Anzeigeeinheit zur Kalibrierung (201) mit Hilfe eines Transfermechanismus';
einen Markierungsanzeigeschritt des Veranlassens der Anzeigeeinheit für Kalibrierung (201), eine Messmarkierung eines ersten Anzeigeaspekts (220), der nicht von der Bildaufnahmeoptik (29b) beeinflusst ist, an einer Bestrahlungsposition anzuzeigen, auf die das Hilfsmesslicht auftrifft, wann immer der Abstand geändert wird;
einen Markierungsbild-Erfassungsschritt des Erfassens eines Kalibrierbilds, erhalten für den Fall, dass ein Bild der Messmarkierung des ersten Anzeigeaspekts, das von dem Endoskop (12) aufgenommen wird, wobei das Kalibrierbild ein Bild der Messmarkierung eines zweiten Anzeigeaspekts (222) enthält, welcher von der Bildaufnahmeoptik (29b) beeinflusst wird; und
einen Kalibrierschritt des Ausführens einer Kalibrierung bezüglich der Anzeige der Messmarkierung auf einer Beobachtungs-Anzeigeeinheit (18), auf der Grundlage des Bilds der Messmarkierung des zweiten Anzeigeaspekts (222).

4. Kalibrierverfahren nach Anspruch 3,
bei dem der Kalibrierschritt einen Repräsentativ-Punktdatentabellen-Erzeugungsschritt des Extrahierens eines repräsentativen Punkts aus dem Bild der Messmarkierung des zweiten Anzeigeaspekts (222) enthält, um eine Repräsentativpunktdaten-Tabelle zu erzeugen, so dass repräsentative Punktdaten bezüglich des repräsentativen Punkts der Bestrahlungsposition zugeordnet werden.

5. Kalibrierverfahren nach Anspruch 3,
bei dem der Kalibrierschritt einen Markierungstabellen-Erzeugungsschritt des Erzeugens einer Markierungstabelle zum Anzeigen des Bilds der Messmarkierung in dem zweiten Anzeigeaspekt (222) durch die Beobachtungs-Anzeigeeinheit (18) enthält, indem das Bild der Messmarkierung des zweiten Anzeigeaspekts (222) mit der Bestrahlungsposition in Bezug gebracht wird.

6. Kalibrierverfahren nach einem der Ansprüche 3 bis 5,
bei dem der erste Anzeigeaspekt eine Größe oder eine Form entsprechend einer Markierungs-Anzeigeposition ist, und der zweite Anzeigeaspekt eine Größe oder eine Form entsprechend einer Markierungs-Anzeigeposition ist.

## Revendications

1. Dispositif d'étalonnage (200) pouvant être connecté à un appareil d'endoscopie (10) incluant un endoscope (12), lequel émet une lumière de mesure auxiliaire et capture une image d'un sujet éclairé avec la lumière de mesure auxiliaire par le biais d'un système optique de prise de vues (29b), dans lequel la lumière de mesure auxiliaire croise l'axe optique du système optique de prise de vues (29b), le dispositif d'étalonnage (200), comprenant :
un mécanisme de transfert (202) configuré pour maintenir une partie d'extrémité distale (12d) de l'endoscope (12) vers une unité d'affichage (201) pour étalonnage et configuré pour modifier une distance entre la partie d'extrémité distale de l'endoscope (12) et l'unité d'affichage pour étalonnage (201) ;
une unité de commande d'affichage (40) pour étalonnage configurée pour faire en sorte que l'unité d'affichage pour étalonnage (201) affiche un marqueur de mesure d'un premier aspect d'affichage (220), lequel n'est pas affecté par le système optique de prise de vues (29b), sur une position d'irradiation sur laquelle la lumière de mesure auxiliaire est appliquée à chaque fois que la distance est modifiée ;
une unité d'acquisition d'image de marqueur configurée pour acquérir une image d'étalonnage obtenue dans un cas où une image du marqueur de mesure du premier aspect d'affichage (220) affiché sur l'unité d'affichage pour étalonnage (201) est capturée par l'endoscope (12), l'image d'étalonnage incluant une image d'un marqueur de mesure d'un second aspect d'affichage (222) affecté par le système optique de prise de vues (29b), et
une unité d'étalonnage (208) configurée pour réaliser un étalonnage lié à l'affichage du marqueur de mesure sur une unité d'affichage d'observation (18) sur la base de l'image d'étalonnage.

2. Dispositif d'étalonnage selon la revendication 1,
dans lequel l'unité d'étalonnage (208) réalise un traitement d'extraction de point représentatif pour extraire un point représentatif à partir de l'image du marqueur de mesure du second aspect d'affichage (222), et un traitement de création de table pour créer une table de données de points représentatifs, de sorte que des données de points représentatifs concernant le point représentatif sont associées à la position d'irradiation.

3. Procédé d'étalonnage destiné à un appareil d'endoscopie (10) incluant un endoscope (12), lequel émet une lumière de mesure auxiliaire et capture une image d'un sujet éclairé avec la lumière de mesure auxiliaire par le biais d'un système optique de prise de vues (29b), dans lequel la lumière de mesure auxiliaire croise l'axe optique du système optique de prise de vues (29b), le procédé d'étalonnage comprenant les étapes suivantes :
une étape de distance variable pour maintenir une partie d'extrémité distale (12d) de l'endoscope (12) vers une unité d'affichage (201) pour étalonnage et modifier une distance entre la partie d'extrémité distale de l'endoscope et l'unité d'affichage pour étalonnage (201) par un mécanisme de transfert ;
une étape d'affichage de marqueur pour faire en sorte que l'unité d'affichage pour étalonnage (201) affiche un marqueur de mesure d'un premier aspect d'affichage (220), lequel n'est pas affecté par le système optique de prise de vues (29b), sur une position d'irradiation sur laquelle la lumière de mesure auxiliaire est appliquée à chaque fois que la distance est modifiée ;
une étape d'acquisition d'image de marqueur pour acquérir une image d'étalonnage obtenue dans un cas où une image du marqueur de mesure du premier aspect d'affichage affiché sur l'unité d'affichage pour étalonnage est capturée par l'endoscope (12), l'image d'étalonnage incluant une image d'un marqueur de mesure d'un second aspect d'affichage (222) affecté par le système optique de prise de vues (29b), et
une étape d'étalonnage pour réaliser un étalonnage lié à l'affichage du marqueur de mesure sur une unité d'affichage d'observation (18) sur la base de l'image du marqueur de mesure du second aspect d'affichage (222).

4. Procédé d'étalonnage selon la revendication 3,
dans lequel l'étape d'étalonnage inclut une étape de création de table de points représentatifs pour extraire un point représentatif à partir de l'image du marqueur de mesure du second aspect d'affichage (222), et créer une table de données de points représentatifs, de sorte que des données de points représentatifs concernant le point représentatif sont associées à la position d'irradiation.

5. Procédé d'étalonnage selon la revendication 3,
dans lequel l'étape d'étalonnage inclut une étape de création de table de marqueurs pour créer une table de marqueurs pour afficher l'image du marqueur de mesure du second aspect d'affichage (222) par l'unité d'affichage d'observation (18) en associant l'image du marqueur de mesure du second aspect d'affichage (222) à la position d'irradiation.

6. Procédé d'étalonnage selon l'une quelconque des revendications 3 à 5,
dans lequel le premier aspect d'affichage est une taille ou une forme correspondant à une position d'affichage de marqueur, et le second aspect d'affichage est une taille ou une forme correspondant à une position d'affichage de marqueur.
